(19) Europäisches Patentamt
European Patent Office
Office européen des brevets

(11) Publication number: **0 290 239 B1**

(12) # EUROPEAN PATENT SPECIFICATION

(45) Date of publication of patent specification: **09.10.91 Bulletin 91/41**

(51) Int. Cl.$^5$: **C07C 35/21**, C07C 29/20

(21) Application number: **88304033.9**

(22) Date of filing: **04.05.88**

(54) **Process for the hydrogenation of bis-phenols.**

(30) Priority: **05.05.87 IT 2036887**

(43) Date of publication of application: **09.11.88 Bulletin 88/45**

(45) Publication of the grant of the patent: **09.10.91 Bulletin 91/41**

(84) Designated Contracting States: **BE DE ES FR GB IT NL SE**

(56) References cited:
**US-A- 4 001 343**
**US-A- 4 192 960**
**US-A- 4 503 273**
**PATENT ABSTRACTS OF JAPAN, unexamined applications, C section, vol. 2, no. 152, December 20, 1978, THE PATENT OFFICE JAPANESE GOVERNMENT, page 3691 C 78**
**English translation of Japanese Patent Application 53-119855**

(73) Proprietor: **Montedison S.p.A.**
**31, Foro Buonaparte**
**I-20121 Milan (IT)**

(72) Inventor: **Gardano, Andrea**
**11, corso Roma**
**I-13039 Trino Vercellese Vercelli (IT)**
Inventor: **Casagrande, Francesco**
**39, viale G. Cesare**
**I-28100 Novara (IT)**
Inventor: **Foa', Marco**
**19, via del Sabbione**
**I-28100 Novara (IT)**
Inventor: **Petrini, Guido**
**11, via Pasubio**
**I-28066 Galliate Novara (IT)**
Inventor: **Barisone, Riccardo**
**65/16, viale Kennedy**
**I-28100 Novara (IT)**
Inventor: **Chapoy, Lawrence**
**60, via Davicini**
**I-28040 Lesa Novara (IT)**

(74) Representative: **Whalley, Kevin et al**
**MARKS & CLERK 57/60 Lincoln's Inn Fields**
**London WC2A 3LS (GB)**

**EP 0 290 239 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of products of the general formula :

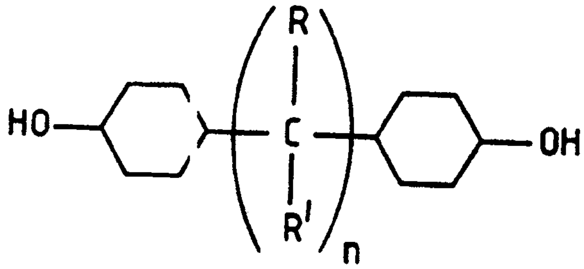

(I')

wherein R and R', which may be the same or different, are hydrogen or $C_1$-$C_{10}$-alkyl radicals and n is 0 or 1, by catalytic hydrogenation of the corresponding aromatic derivatives of the formula :

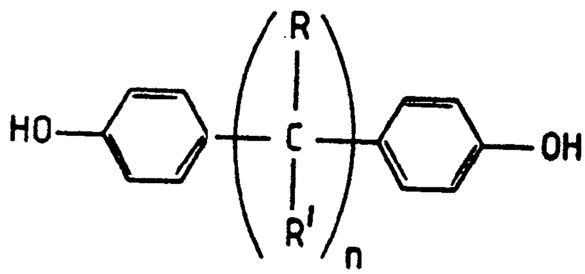

(I)

wherein R, R' and n have the above specified meaning.

Products of formula (I') wherein n = 1 are known compounds, generally used as comonomers for the preparation of thermosetting polyester resins, and namely when both substituents R and R' are methyl groups.

It is also known that for these products at least three stereoisomeric forms are possible, which differ among them as to the reciprocal position of the alcoholic hydroxyl groups with reference to the radical —C(RR')—, generally in equatorial position in respect of the two cyclohexane rings.

In particular, the trans-trans stereoisomer, which shows both the hydroxyl groups in equatorial position, has been used to obtain high molecular weight polyesters showing good mechanical properties (Journal of polymer Science, vol. 24, pages 419-426, 1986).

Compounds of formula (I') wherein n = 0 showing trans-trans stereoisomery are used as intermediate compounds for the synthesis of liquid crystals.

Therefore, it is desirable from an industrial point of view, to provide a process for the preparation of compounds of general formula (I') which allows the obtaining of an isomeric mixture rich in the trans-trans isomer.

Furthermore, it would also be desirable if such a process may allow the obtaining of the above mentioned products with a high purity degree to reduce as much as possible the presence of monofunctional derivatives which stop the growth of polymer chains in condensation reactions and represent impurities which can be removed only with difficulty.

A process for the preparation of products having the general formula (I') wherein n = 1 is described in U.S. Patent No. US-A-4,503,273 ; according to this patent the mentioned products can be obtained by hydrogenation of the corresponding aromatic derivatives in the presence of a catalytic system preferably consisting of nickel borne on a solid acidic diluent such as silica, alumina, or silica-alumina. A promoter of the catalytic system is a combination of a non-aqueous organic solvent with a base selected from oxides, hydroxides and carbonates of alkali and alkaline-earth metals.

The drawback of this process, and in particular of the corresponding catalytic system, is that the hydrogenation reaction has a selectivity as to useful hydrogenated product lower than 95% by mols, with a maximum ratio among the trans-trans isomers and cis-cis and cis-trans isomers about equal to 1, or in some cases lower than 1.

Furthermore, the highest yield as to trans-trans isomer is obtained only by adversely affecting the reaction selectivity.

The presence of impurities, in particular of mono-hydroxylated derivatives, makes it necessary to purify the reaction mixture in order to use it in the synthesis of high molecular weight polyesters.

Similar problems have been found with catalysts consisting of ruthenium borne on carbon, nickel on diatomyte, or Raney-nickel as described in Japanese Patent Application No. 78/119855. In fact, by the use of the above mentioned catalysts, there are obtained either low selectivity as to the trans-trans isomer when ruthenium catalyst is used, or selectivity as to useful hydrogenated product lower than 90% when nickel catalysts are used.

Products of formula (I') wherein n = 0 are described in "Journal of American Chemical Society" vol. 76, page 1733 (1954) and are prepared by hydrogenation of the corresponding bis-phenol with Raney-nickel catalysts, but unsatisfactory reaction yields are obtained.

We have found that products having the general formula (I') with a high purity degree and a trans-trans isomer content higher than 55% by weight can be obtained by a hydrogenation process of the corresponding hydroxy substituted aromatic derivatives, which process uses a hydrogenation catalyst borne on activated carbon.

The present invention provides a process for the hydrogenation of bis-phenols of formula (I), comprising reacting the bis-phenol with hydrogen in the presence of a catalytic system which comprises palladium on activated carbon.

Examples of bis-phenols to be treated by the process of the invention are : 4,4'-dihydroxydiphenyl, 1,1-bis (4-hydroxyphenyl) ethane,
2,2-bis(4-hydroxyphenyl)-propane,
2,2-bis(4-hydroxyphenyl)butane, and
3,3-bis(4-hydroxyphenyl)hexane.

Preferred products are :
2,2-bis(4-hydroxyphenyl)propane and
4,4-dihydroxydiphenyl.

The activated carbon used as supporting material for palladium is a known product and has a specific surface less than 1000 $m^2/g$, in particular from 400 to 900 $m^2/g$, more preferably from 600 to 800 $m^2/g$.

The catalytic system can be prepared by one of the general methods which can be found in the literature.

For instance, an acid solution of palladium halide may be added to an alkaline suspension of powdered activated carbon, the size of the particles being, at least for 80% b.w., less than 40 micrometers.

After having completed the addition, the hydrolysis compounds deposited on the carbon are transformed into metal by treatment with a reducing agent at a temperature from 20 to 90°C. Reducing agents particularly suitable for this purpose are sodium hypophosphite and sodium formate.

The solid product is then recovered by filtration and washed with water at a temperature from 20 to 100°C until halides are removed.

The final catalyst, which contains about 50% b.w. of water, can be used as such or can be previously dried.

According to a preferred embodiment of the process of the present invention, the thus obtained catalytic system preferably contains a small amount of alkali so as to provide a pH higher than 7 if dispersed in water. For this reason, it is preferable to treat the final catalyst with solutions of salts of alkali or alkaline-earth metals.

The content of palladium in the catalytic system is not critical and is preferably from 1 to 20% by weight, calculated on the dry catalyst. However, concentrations higher than 20% by weight can be used.

The hydrogenation reaction can occur in bulk or in a solvent optionally added with water. As solvent an organic solvent is preferably used, selected from esters, ethers, alcohols, saturated or cyclic having boiling point higher than 50°C, saturated or cyclic hydrocarbons containing from 5 to 20 carbon atoms, etc.

Particularly suitable have proved to be ethyl acetate, methyl-t-butylether and ethanol, either alone or added with water up to 2% by volume and cyclohexane.

The reaction temperature is preferably from 80 to 160°C, and the hydrogen pressure is preferably from 50 to 150 atm, more preferably from 80 to 120 atm.

The process of the present invention allows products to be obtained having the general formula (I') with a high purity degree, in that reaction selectivity is higher than 99% by weight, and with a trans-trans isomer content higher than 55% by weight, generally higher than 60%.

The invention will be further described with reference to the following illustrative Examples.

## EXAMPLE 1

Into a 1 liter stainless steel autoclave were introduced 40 g of 2,2-bis(4-hydroxyphenyl)propane (Bisphenol A), 500 ml of ethyl acetate and 4 g of catalyst consisting of activated carbon having a specific surface area of about 750 $m^2/g$, containing 50% b.w. of water and bearing 5% b.w. of palladium calculated on the dry catalyst.

After closing the autoclave and washing with nitrogen to expel the air, 80 atm of hydrogen were introduced. By keeping the autoclave under stirring, the temperature was raised to 140°C and the pressure rose to 100 atm.

The whole was kept at 140°C for 12 hours, by replacing the hydrogen adsorbed in order to maintain a pressure of 100 atm. After having cooled to room temperature, the remaining pressure was discharged and the autoclave was washed with nitrogen.

The suspension was discharged and the whole was completely dissolved by adding 500 ml of ethanol.

The whole was filtered on paper to recover the catalyst which was washed with ethanol. The clear solution was evaporated thus obtaining 43.2 g of hydrogenated bisphenol A with a 99.7% yield (conversion 100%, selectivity 99.7%).

By gas chromatographic analysis of the reaction crude product (capillary column of molten silica bonded phase SPB1 30 M 1.0 um 0.32 mm ID SUPELCO INC.) the isomeric composition of the hydrogenated bisphenol A was found to be as follows : 62.6% trans-trans ; 32.5% cis-trans ; 4.9% cis-cis.

EXAMPLE 2

The procedure of example 1 was followed, but using as substrate 4,4'-dihydroxydiphenyl and using ethyl acetate containing 1% of water as solvent. 42.4 g of 4,4-dihydroxydicyclohexyl were obtained with 99.5% yield (conversion 100%, selectivity 99.5%). By gas chromatographic analysis of the reaction crude product the isomeric composition was found to be as follows : 60.2% trans-trans ; 34.6% cis-trans ; 5.2% cis-cis.

EXAMPLE 3

The catalyst used in example 1 was dried at 110°C until constant weight. 2 g of this catalyst was used by working under the same conditions as in example 1. Hydrogenated bisphenol A was obtained with a practically quantitative yield (higher than 99.5%). By gas chromatographic analysis of the reaction crude product, the isomeric composition was found to be as follows : 57.2% trans-trans ; 36.3% cis-trans ; 6.5% cis-cis.

EXAMPLE 4

The procedure of example 1 was followed, but using a catalyst (palladium on 5% activated carbon containing 50% of water) prepared using carbon having a specific surface area of about 600 $m^2/g$. Hydrogenated bisphenol A was obtained with practically quantitative yield (higher than 99.5%). By gas chromatographic analysis of the reaction crude product, the isomeric composition was found to be as follows : 56.0% trans-trans; 37.2% cis-trans ; 6.8% cis-cis.

EXAMPLE 5

The procedure of example 1 was followed, but using a catalyst (palladium on 5% carbon containing 50% water) washed with acid to remove ash and thereafter washed again with an aqueous solution containing 1.5% of sodium carbonate. Hydrogenated bisphenol A was obtained with practically quantitative yield (higher than 99.5%). By gas chromatographic analysis of the reaction crude product, the isomeric composition was found to be as follows : 63.2% trans-trans ; 31.7% cis-trans ; 5.1% cis-cis.

EXAMPLE 6

The procedure of example 1 was followed, but using as solvent cyclohexane and thus obtaining hydrogenated bisphenol A with practically quantitative yield (higher than 99.5%). By gas chromatographic analysis of the reaction crude product, the isomeric composition was found to be as follows : 56.8% trans-trans ; 36.1% cis-trans ; 7.1% cis-cis.

EXAMPLE 7

The procedure of example 1 was followed, but using ter.butyl-methyl ether as solvent. Hydrogenated bisphenol A was obtained having the following isomeric composition (gas chromatographic analysis of the reaction crude product) : 58.8% trans-trans ; 33.3% cis-trans ; 6.9% cis-cis.

EXAMPLE 8

The procedure of example 1 was followed, but using ethanol as solvent. Hydrogenated bisphenol A was obtained having the following isomeric composition (gas chromatographic analysis of the reaction crude product) : 59.8% trans-trans ; 34.7% cis-trans ; 5.5% cis-cis.

EXAMPLE 9

The procedure of example 1 was followed, but using a catalyst (palladium on 5% carbon containing 50% water) basic washed with a solution containing 1.5% b.w. of sodium carbonate. Hydrogenated bisphenol A was obtained having the following isomeric composition (gas chromatographic analysis of the reaction crude product) : 65.4% trans-trans ; 30.1% cis-trans ; 4.5% cis-cis.

## Claims

1. A process for the hydrogenation of bisphenols having the formula :

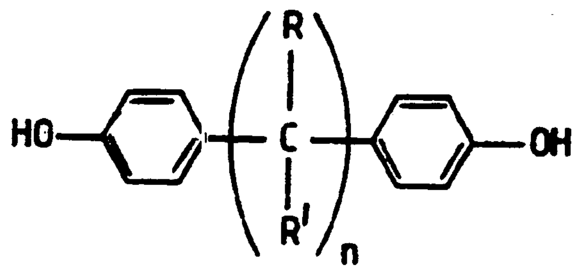

(I)

wherein R and $R_1$, which may be the same or different from each other, are hydrogen or $C_1$-$C_{10}$ alkyl radicals, and n is 0 or 1, to products having the formula :

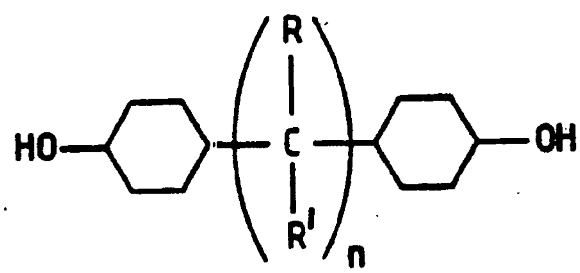

wherein R, $R_1$ and n have the same meanings as defined above, said process being characterized in that a bisphenol of the formula (I) is reacted with hydrogen in the presence of a catalytic system which is palladium supported on activated carbon.

2. A process as claimed in Claim 1, characterized in that the activated carbon has a specific surface area less than 1000 $m^2/g$.

3. A process as claimed in Claim 2, characterized in that the activated carbon has a specific surface area from 400 to 900 $m^2/g$, preferably from 600 to 800 $m^2/g$.

4. A process as claimed in any of Claims 1 to 3, characterized in that the catalytic system has a pH higher than 7 in water.

5. A process as claimed in any of Claims 1 to 4, characterized in that the content of palladium in the catalytic system is from 1 to 20% by weight calculated on the dry catalyst.

6. A process as claimed in any of Claims 1 to 5, characterized in that the reaction temperature is from 80 to 160°C and in that the hydrogen pressure is from 50 to 150 atm, preferably from 80 to 120 atm.

7. A process as claimed in any of Claims 1 to 6, characterized in that the bisphenol is 2,2-bis(4-hydroxyphenyl)propane or 4,4'-dihydroxydiphenyl.

8. A process as claimed in any of Claims 1 to 7, characterized in that the hydrogenation reaction is carried out in bulk or in solvent.

9. A process as claimed in Claim 8, characterized in that the solvent is selected from ethyl acetate, methyl-tert.butyl-ether and ethanol, either alone or added with water up to 2% by volume and cyclohexane.

## Patentansprüche

1. Verfahren zum Hydrieren von Bisphenolen mit der Formel :

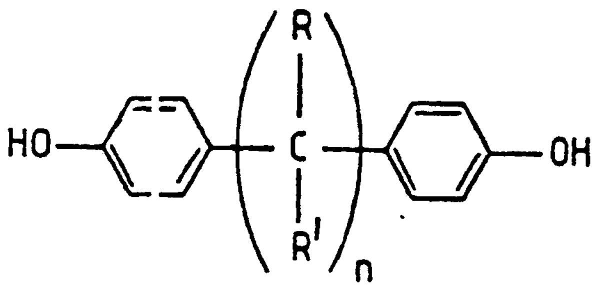

(I)

worin R und $R_1$, die gleich oder verschieden voneinander sein können, Wasserstoff oder $C_1$-$C_{10}$ Alkylradikale sind, und n 0 oder 1 ist, zu Produkten mit der Formel :

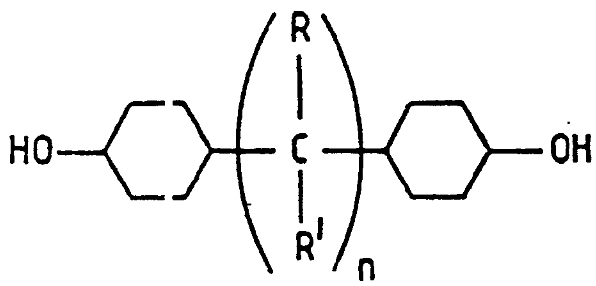

worin R, $R_1$ und n dieselbe Bedeutung wie oben haben, und das Verfahren dadurch gekennzeichnet ist, daß ein Bisphenol der Formel (I) mit Wasserstoff in Gegenwart eines katalytischen Systems aus Palladium auf einen Aktivkohleträger umgesetzt wird.

2. Verfahren nach Anspruch 1, dadurch gekennzeichnet, daß die Aktivkohle eine spezifische Oberfläche von weniger als 1.000 $m^2/g$ hat.

3. Verfahren nach Anspruch 2, dadurch gekennzeichnet, daß die Aktivkohle eine spezifische Oberfläche von 400 bis 900 $m^2/g$, vorzugsweise von 600 bis 800 $m^2/g$ hat.

4. Verfahren nach einem der Ansprüche 1 bis 3, dadurch gekennzeichnet, daß das katalytische System in Wasser einen pH-Wert von größer 7 hat.

5. Verfahren nach einem der Ansprüche 1 bis 4, dadurch gekennzeichnet, daß der Palladiumgehalt im katalytischen System 1 bis 20 Gew.%, berechnet auf den trockenen Katalysator, beträgt.

6. Verfahren nach einem der Ansprüche 1 bis 5, dadurch gekennzeichnet, daß die Reaktionstemperatur 80 bis 160°C ist und daß der Wasserstoffdruck 50 bis 150 atm, vorzugsweise 80 bis 120 atm ist.

7. Verfahren nach einem der Ansprüche 1 bis 6, dadurch gekennzeichnet, daß das Bisphenol 2,2-bis(4-Hydroxyphenyl)propan oder 4,4'-Dihydroxydiphenyl ist.

8. Verfahren nach einem der Ansprüche 1 bis 7, dadurch gekennzeichnet, daß die Hydrierungsreaktion in Anwesenheit oder Abwesenheit eines Lösungsmittels durchgeführt wird.

9. Verfahren nach Anspruch 8, dadurch gekennzeichnet, daß das Lösungsmittel ausgewählt ist aus Ethylacetat, Methyl-tert.butyl-ether und Ethanol, Ether allein oder unter Zusatz von Wasser bis zu 2 Vol.% und Cyclohexan.

## Revendications

1. Un procédé d'hydrogénation de bisphénols de formule :

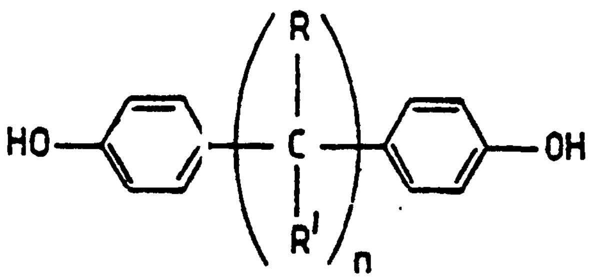

(I)

dans laquelle :

R et $R_1$, qui peuvent être identiques ou différents l'un de l'autre, sont un atome d'hydrogène ou des radicaux alkyles en $C_1$-$C_{10}$, et n est 0 ou 1, en produits de formule :

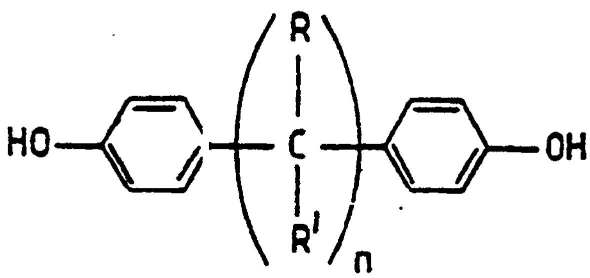

(I)

dans laquelle :

R, $R_1$ et n ont la même signification que celle définie ci-dessus, ledit procédé étant caractérisé en ce qu'un bisphénol de formule (I) est mis à réagir avec de l'hydrogène en présence d'un système catalytique consistant en du palladium supporté sur du charbon actif.

2. Un procédé selon la revendication 1, caractérisé en ce que le charbon actif a une aire surfacique spécifique inférieure à 1000 $m^2/g$.

3. Un procédé selon la revendication 2, caractérisé en ce que le charbon actif a une aire surfacique spécifique de l'ordre de 400 à 900 $m^2/g$, de préférence de 600 à 800 $m^2/g$.

4. Un procédé selon l'une quelconque des revendications 1 à 3, caractérisé en ce que le système catalytique présente un pH supérieur à 7 dans l'eau.

5. Un procédé selon l'une quelconque des revendications 1 à 4, caractérisé en ce que la teneur en palladium du système catalytique est de l'ordre de 1 à 20% en poids calculé par rapport au catalyseur sec.

6. Un procédé selon l'une quelconque des revendications 1 à 5, caractérisé en ce que la température réactionnelle est de l'ordre de 80 à 160°C, et en ce que la pression d'hydrogène est de l'ordre de 50 à 150 atm., de préférence de 80 à 120 atm.

7. Un procédé selon l'une quelconque des revendications 1 à 6, caractérisé en ce que le bisphénol est du 2,2-bis(4-hydroxyphényl)propane ou du 4,4'-dihydroxydiphényl.

8. Un procédé selon l'une quelconque des revendications 1 à 7, caractérisé en ce que la réaction d'hydrogénation est une réaction en masse ou dans un milieu solvant.

9. Un procédé selon la revendication 8, caractérisé en ce que le solvant consiste en acétate d'éthyle, méthyl-tert.-butyl-éther ou éthanol, soit seul, soit additionné d'eau jusqu'à 2% par volume et de cyclohexane.